Europäisches Patentamt

European Patent Office

Office européen des brevets

(11) Veröffentlichungsnummer: **0 324 729 A1**

(19)

# EUROPÄISCHE PATENTANMELDUNG

(12)

(21) Anmeldenummer: **89890006.3**

(22) Anmeldetag: **10.01.89**

(51) Int. Cl.⁴: **A 61 L 2/04**

(30) Priorität: **12.01.88 AT 50/88**

(43) Veröffentlichungstag der Anmeldung:
**19.07.89 Patentblatt 89/29**

(84) Benannte Vertragsstaaten:
**AT BE CH DE ES FR GB IT LI LU NL SE**

(71) Anmelder: **IMMUNO Aktiengesellschaft für
chemisch-medizinische Produkte
Industriestrasse 72
A-1220 Wien (AT)**

(72) Erfinder: **Eibl, Johann, Dr.
Gustav Tschermakgasse 2
A-1180 Wien (AT)**

**Schwarz, Otto, Dr.
Celtesgasse 5
A-1190 Wien (AT)**

**Elsinger, Fritz, Dr.
Eduard Kleingasse 29
A-1130 Wien (AT)**

**Wöber, Günter, Prof.Dr.
Carolusstrasse 23
A-2522 Oberwaltersdorf (AT)**

**Philapitsch, Anton
Hans Kudlichgasse 5
A-2490 Ebenfurth (AT)**

**Linnau, Yendra, Dr.
Lavendelweg 24
A-1224 Wien (AT)**

**Dorner, Friedrich, Prof.Dr.
Peterlinigasse 17
A-1230 Wien (AT)**

**Trambauer, Karl
Steudelgasse 37-39/1/8
A-1100 Wien (AT)**

**Frechinger, Wolfgang
Nordmanngasse 22
A-1210 Wien (AT)**

(74) Vertreter: **Wolfram, Gustav, Dipl.-Ing.
Schwindgasse 7 P.O. Box 205
A-1041 Wien (AT)**

(54) Verfahren zur Inaktivierung von vermehrungsfähigen filtrierbaren Krankheitserregern in Blutplasmaprodukten.

(57) Es wird ein Verfahren zur Inaktivierung von vermehrungsfähigen filtrierbaren Krankheitserregern in Blutplasmaprodukten, wie Präparationen von Gerinnungsfaktoren, beschrieben, wobei diese Blutplasmaprodukte im festen Zustand in einem geschlossenen Inaktivierungsbehälter in Gegenwart von bestimmten Mengen an Wasser und organischen Verbindungen hitzebehandelt werden, mit der Maßgabe, daß der Wasseranteil zum größten Teil an den Blutplasmaprodukten physikalisch gebunden vorhanden ist, während die organische Verbindung in Gasform in der Atmosphäre vorhanden ist.

Der Wassergehalt der Blutplasmaprodukte beträgt 0,08 bis 0,40, die Konzentration der organischen Vebindungen in der Gasphase des Behälters 0,01 bis 10 g pro Liter Behältervolumen.

EP 0 324 729 A1

**Beschreibung**

## Verfahren zur Inaktivierung von vermehrungsfähigen filtrierbaren Krankheitserregern in Blutplasmaprodukten

Die Erfindung betrifft ein Verfahren zur Inaktivierung von vermehrungsfähigen filtrierbaren Krankheitserregern in Blutplasmaprodukten unter Anwendung erhöhter Temperatur.

Für Inaktivierungsverfahren dieser Art ist eine umfangreiche Literatur vorhanden, wobei diese u.a. umfaßt:
- Erhitzen der Blutprodukte in wässeriger Lösung, gegebenenfalls unter Zusatz viruzider Substanzen, in Anwesenheit von Stabilisatoren,
- Erhitzen der Blutprodukte in trockenem Zustand.

So beschreibt beispielsweise die DE-A - 29 16 711 ein Verfahren zur Behandlung von Gerinnungsfaktoren enthaltenden Präparationen in wässeriger Lösung unter Anwendung einer Temperatur von 30 bis 100°C, wobei der Lösung der Gerinnungsfaktoren eine Aminosäure und ein Mono-, Oligosaccharid oder Zuckeralkohol zugesetzt werden.

In der EP-A2 - 0 035 204 wird ein Verfahren zur Inaktivierung von wässerigen Proteinlösungen, die Faktor VIII, Fibronectin, Globulin, Fibrinogen und andere Proteine enthalten können, beschrieben, wobei die Komposition mit einem Polyol gemischt und die Mischung auf eine Temperatur von 60 bis 75°C erhitzt wird.

In der US-A - 4,379,085 ist ein Verfahren zur Hitzeinaktivierung eines Plasmaproteins, wie $C_1$-Inhibitor oder Faktor IX, in wässeriger Lösung in Gegenwart von Kalium- oder Ammoniumcitrat beschrieben.

In der EP-A2 - 0 077 870 ist ein Virus-Inaktivierungsverfahren beschrieben, bei welchem eine wässerige, Faktor VIII enthaltende Lösung mit Aminosäuren, Monosacchariden, Oligosacchariden, Zuckeralkoholen und Kohlenwasserstoff- oder Hydroxylkohlenwasserstoffcarbonsäuren mit 3 bis 10 Kohlenstoffatomen auf eine Temperatur von 50 bis 80°C erhitzt wird.

Die US-A - 3,041,242 beschreibt die Erhitzung von trockenem, menschlichem Plasma gefolgt von der Einwirkung eines abtötenden Gases im Hochvakuum mit dem Ziel der Virusinaktivierung.

Die DE-A - 34 34 472 und die EP-A2 - 0 240 750 beschreiben die Pasteurisation von pulverförmigen, lyophilisierten Plasmaproteinen in Suspension in einem flüssigen Wärmeträger, u.a. Alkohol und Fettsäureestern. Der Wassergehalt des Systems soll jedoch kleiner als 0,01 sein, und eine allfällige Wirkung dieser Maßnahme auf humanpathogene Viren wird nicht mitgeteilt.

Auch die US-A - 4,490,361 beschreibt die Erhitzung der Suspension eines trockenen Proteinpulvers in einer organischen Flüssigkeit.

Purcell beschreibt in der PCT-Veröffentlichung WO 85/05273 die Inaktivierung lipidhaltiger Viren im trockenen Zustand mit wasserhaltigen Halogenkohlenwasserstoffen. Aufgrund der geringen Löslichkeit von Wasser in Chloroform, dem bevorzugten Halogenkohlenwasserstoff, beträgt auch hier der Wassergehalt des Systems weniger als 0,05.

Die EP-A2 - 0 094 611 beschreibt ein Verfahren zur Behandlung einer Faktor VIII enthaltenden Komposition im trockenen Zustand mit weniger als 0,05 (5 Gew.%) Wasser, unter Anwendung einer Temperatur von wenigstens 60°C zwecks Inaktivierung von vorhandenen Hepatitis-Viren.

Die veröffentlichte PCT-Anmeldung WO 82/03871 beschreibt ein Verfahren zur Behandlung von Blutgerinnungsenzyme enthaltenden Zubereitungen, wobei diese zur Inaktivierung vorhandener infektiöser Viren im trockenen Zustand erhitzt werden; als trockener Zustand wird ein solcher mit weniger als 0,05 (5 Gew.%) Wasser definiert.

In der US-A - 4,640,834 der Anmelderin ist darüber hinaus ein Inaktivierungsverfahren beschrieben, bei welchem die Blutprodukte in festem Zustand auf einen Gehalt an Wasser, Methanol oder Äthanol von mehr als 0,05 (5 Gew.%) und weniger als 0,70 (70 Gew.%), vorzugsweise weniger als 0,40 40 Gew.%), eingestellt und in einem geschlossenen Behälter bei einer Temperatur im Bereich von 50 bis 121°C unter Erhöhung des Partialdampfdruckes des Wassers, Methanols oder Äthanols behandelt werden.

Nach einem weiteren Stand der Technik ist aus der EP-B1 - 0 053 338 die Hitzebehandlung einer wässerigen Lösung der Blutgerinnungsfaktoren IX und X in Anwesenheit von Saccharose und Glycin bekannt, wobei die biologische Aktivität dieser Gerinnungsfaktoren durch Zusatz von 0,05 bis 2 M Calciumionen stabilisiert werden soll.

Auch die EP-A - 0 137 428 bezieht sich auf ein Verfahren zur Herstellung einer Präparation der Blutgerinnungsfaktoren II, VII, IX und X durch Erhitzen in wässeriger Lösung unter Zusatz von Saccharose, Glycin, Calciumionen (1 bis 50 mM) und einem Chelatbildner.

Allen diesen Verfahren ist das Bestreben gemeinsam, die potentielle Infektivität der Blutplasmaprodukte zu beseitigen, ihre biologische Aktivität aber weitgehend zu erhalten. Im Falle der Inaktivierung von hitzestabilen Viren, wie z.B. Hepatitis-Viren, ist dies bisher nicht zufriedenstellend gelungen.

Die vorliegende Erfindung stellt sich die Aufgabe, die bisherigen Schwierigkeiten zu überwinden und eine Inaktivierungsmethode zu schaffen, bei welcher unter vergleichbarer Inaktivierungszeit und Inaktivierungstemperatur eine größere Wirksamkeit der Virusinaktivierung und dadurch eine größere Sicherheit gegen Übertragung von pathogenen Viren erreicht wird; gleichzeitig soll die Erhaltung der biologischen Aktivität des jeweiligen Blutplasmaproduktes weitgehend sichergestellt sein.

Die Erfindung geht aus von der US-A - 4,640,834 und stellt insoferne eine Verbesserung und Weiterentwicklung dar, als gefunden wurde, daß eine bestimmte Kombination eines wässerigen und organischen Beandlungsmediums die besten Ergebnisse ergibt.

Dementsprechend besteht die Erfindung bei einem Verfahren zur Inaktivierung von vermehrungsfähigen filtrierbaren Krankheitserregern in Blutplasmaprodukten unter Anwendung erhöhter Temperatur, wobei die Blutplasmaprodukte in festem Zustand in einem geschlossenen Inaktivierungsbehälter in Gegenwart hydroxylgruppenhältiger Verbindungen hitzebehandelt werden, deren Konzentration bezogen auf die Blutplasmaprodukte mehr als 0,05 (5 Gew.%) beträgt, in der Kombination der folgenden Maßnahmen:

- daß die Blutplasmaprodukte vor der Hitzebehandlung auf einen Wassergehalt von 0,08 bis 0,40 (8 bis 40 Gew.%) eingestellt werden - wobei das im System enthaltene Wasser während der Hitzebehandlung im wesentlichen physikalisch an den Blutplasmaprodukten gebunden bleibt - und

- daß während der Hitzebehandlung der Blutplasmaprodukte in der Gasphase des Behälters eine organische Verbindung aus der Reihe Äthanol, Essigsäureethylester, Diethylether, Dimethylformamid, Toluol, Chloroform, n-Heptan, 1,2-Diacetoxiethan oder Aceton mit einer Konzentration von 0,01 bis 10 g der organischen Verbindung pro Liter Behältervolumen vorgesehen wird.

Mit Vorteil wird die Hitzebehandlung in Gegenwart von Calciumionen durchgeführt, wobei die Calciumionen entweder in irgendeiner Stufe des Fraktionierungsprozesses der Blutplasmaprodukte vor ihrer Lyophilisierung zugefügt werden können oder das lyophilisierte Produkt mit Calciumionen enthaltendem Wasser befeuchtet wird. Die Zugabe der Calciumionen hat eine verbesserte Stabilisierung der Aktivitäten der Blutplasmaprodukte zur Folge.

Eine bevorzugte Ausführungsform des erfindungsgemäßen Verfahrens besteht darin, daß die Blutplasmaprodukte mit Wasserdampf befeuchtet werden, bis der gewünschte Feuchtigkeitsgehalt erreicht ist, die Blutplasmaprodukte in den Inaktivierungsbehälter eingebracht werden und anschließend die organische Verbindung in Gasform in den Behälter eingeleitet wird, um die gewünschte Konzentration einzustellen, worauf der geschlossene Behälter mit den Blutplasmaprodukten auf die Inaktivierungstemperatur gebracht und während der für die Virusinaktivierung erforderlichen Zeit erhitzt wird.

Vorteilhaft wird die Befeuchtung der Blutplasmaprodukte mit dem Wasserdampf unter Vakuum durchgeführt.

Die Hitzebehandlung wird vorzugsweise bei einer Temperatur von 50 bis 80°C durchgeführt.

Nach einer bevorzugten Ausführungsform wird die Behälteratmosphäre - vor der Einleitung der organischen Verbindung - durch Verdrängung mit inertem Gas, insbesondere mit trockenem Stickstoff, sauerstofffrei gemacht.

Das erfindungsgemäße Verfahren wird durch die folgenden Beispiele näher erläutert:

Beispiel 1:

Es wurde eine Faktor VIII-Präparation nach der in der AT-B - 379 510 beschriebenen Methode hergestellt und gefriergetrocknet. 350 g des lyophilisierten Bulkpulvers, welches einen Feuchtigkeitsgehalt von 0,019 aufwies, wurden in einen evakuierbaren und mit einer Wasserdampfzuführung ausgerüsteten Behälter eingebracht und der Behälter evakuiert, wobei sich ein Druck von 4 mbar einstellte. 90 ml destilliertes Wasser - entsprechend einem Feuchtigkeits-soll-Wert von 0,2 (20 Gew.%) - wurden in Dampfform zur Befeuchtung des Pulvers in den Behälter eingeleitet, wobei der Druck auf 33 mbar anstieg. Nach kurzer Einwirkungszeit des Wasserdampfes auf das lyophilisierte Material ergab sich ein Gleichgewichtsdruck von 23 mbar.

Der Befeuchtungsbehälter wurde sodann mit trockenem Stickstoff gespült und auf Atmosphärendruck gebracht. Die Gewichtszunahme des Pulvers betrug 85,5 g. Das befeuchtete Pulver wurde 24 Stunden lang äquilibriert und eine Wassergehaltsbestimmung nach Karl Fischer (Scholz E., Z. analyt. Chem. 314, 567-571 (1983)) vorgenommen, wobei der Wassergehalt von drei aliquoten Proben mit 0,203 / 0,200 / 0,202 festgestellt wurde.

Eine Teilmenge von 162 g des so vorbereiteten befeuchteten Bulkpulvers wurde in einen verschließbaren, evakuierbaren und mit Gaszuleitungen versehenen Inaktivierungsbehälter (Volumen 15,4 l) eingebracht und zweimal nacheinander auf einen Druck von 100 mbar evakuiert; dann wurde ein Druckausgleich auf Umgebungsdruck mit trockenem Stickstoff vorgenommen. Anschließend wurde der Druck im Inaktivierungsapparat erneut auf 100 mbar gesenkt und durch eine Gaszuleitung 3,85 g Äthanol in Dampfform eingeleitet; dies entspricht einer Menge von 0,25 g Äthanol/l Gesamtvolumen des Inaktivierungsbehälters.

Anschließend wurde der Druck im Inaktivierungsbehälter mit trockenem Stickstoff auf Umgebungsdruck erhöht, der Behälter geschlossen und 10 Stunden lang auf 60°C erhitzt. Während der Hitzebehandlung wurde im Behälter ein Gesamtdruck von 1222 mbar und ein Taupunkt von 49,8°C gemessen.

Aus der Bestimmung des Taupunktes ergibt sich der Rückschluß, wie das Wasser beim Erhitzen des feuchten Pulvers auf 60°C zwischen dem Festkörper (Lyophilisat) und der Gasphase im Inaktivierungsbehälter verteilt ist. Eine direkte Messung des Wassergehaltes des Pulvers bei 60°C ist nicht möglich, ohne die Gleichgewichtseinstellung im geschlossenen System nachhaltig zu stören. Jedoch läßt sich aus der Messung des Taupunktes in der Gasphase des geschlossenen Inaktivierungsbehälters bei 60°C der Wassergehalt der Atmosphäre und daraus der des Lyophilisates errechnen. Dabei hat sich gezeigt, daß das Faktor VIII-hältige Material auch beim Erhitzen auf 60°C etwa 96 % des Wassergehaltes in Form von physikalisch gebundenem Wasser enthält.

Im einzelnen kann die Berechnung mit Hilfe der in Tabelle I angegebenen Daten durchgeführt werden:

EP 0 324 729 A1

Tabelle I

Wassergehalt von feuchtem, Faktor VIII-hältigem Lyophilisat beim Erhitzen auf 60°C

| 1 | 2 | 3 | 4 | 5 | 6 | 7 | 8 |
|---|---|---|---|---|---|---|---|
| Faktor VIII- Lyoph Lisat <br><br> | Masse des feuchten Produktes <br><br> g | g Wasser pro g feuchtes Produkt <br><br> | Gesamtmenge Wasser im System <br><br> g | Volumen des Inaktivie- rungsgefäßes <br><br> l | Tempera- tur <br><br><br> °C/K | Gesamt- druck $P_{tot}$ <br><br> mbar | Taupunkt (TP) <br><br><br> °C |
|  | 162 | 0,20 | 32,4 | 15,4 | 60/333 | 1222 | 49,8 |

| 9 | 10 | 11 | 12 | 13 | 14 | 15 |
|---|---|---|---|---|---|---|
| $P_{H2O}/TP$ Sättigungs- dampfdruck beim Taupunkt <br><br><br><br> mbar | $P_{N2/60}$ (Sp.7-9) <br><br><br><br><br> mbar | $\dfrac{g_{H_2O}\ (Gas)}{g_{N2}}$ (Gleichung 1) <br><br> $0{,}643 \cdot \dfrac{Sp.9}{Sp.10}$ | $V_{1gN2/60}$ (Gleichung 3) <br><br> $2{,}97 \cdot \dfrac{Sp.6\ K}{Sp.10}$ <br><br> l | $\dfrac{g_{H_2O}\ (Gas)}{l_{N2/60}}$ <br><br> $\dfrac{Sp.11}{Sp.12}$ | $H_2O$ in der Inaktivie- rungsgefäß <br><br><br> Sp.13.Sp.5 <br><br> g | Gasphase % vom Gesamt- wasser <br> $\dfrac{Sp.14}{Sp.4} \cdot 100$ |
| 122 | 1100 | 0,0713 | 0,8991 | 0,0793 | 1,22 | 3,8 |

Der Wassergehalt des befeuchteten Pulvers in diesem Beispiel betrug 0,20 gemäß der Bestimmung nach Karl Fischer (Spalte 3 der Tabelle I).

Während der anschließenden Behandlung des feuchten Pulvers unter Stickstoffatmosphäre bei 60°C in dem geschlossenen Inaktivierungsbehälter wurde ein Gesamtdruck von 1222 mbar (Spalte 7) und der Taupunkt von 49,8°C (Spalte 8) gemessen. Als Meßgerät wurde ein Taupunkt Hygrometer, Modell 660, der EG & G Environmental Equipment verwendet. Aus den hygrometrischen und psychrometrischen Tabellen der Smithsonian Institution kann man den Sättigungsdampfdruck $p_{H_2O/TP}$ von Wasser beim gemessenen Taupunkt ablesen (Spalte 9). Durch Subtraktion dieses Sättigungsdruckes $p_{H_2O/TP}$ vom Gesamtdruck $p_{tot}$ erhält man den Partialdruck von Stickstoff bei 60°C $p_{N_2/60}$ (Spalte 10 = Spalte 7 -Spalte 9). Mit Hilfe dieser Werte kann man den Ausdruck $g_{H_2O}$ pro $g_{N_2}$ (Spalte 11) in der Gasphase nach folgender Formel berechnen:

$$\frac{g_{H_2O} \ (Gas)}{g_{N_2}} = \frac{MG \ H_2O}{MG \ N_2} \cdot \frac{p_{H_2O/TP}}{p_{N_2}} = 0,643 \cdot \frac{p_{H_2O/TP}}{p_{N_2}} \qquad \text{Gleichung 1}$$

Zur Umformung des Ausdruckes der Spalte 11 in den anschaulichen Ausdruck $g_{H_2O}$ (Gas) pro Liter $N_2$ wird der Wert in Spalte 11 durch das Volumen von 1 g $N_2$ unter den Bedingungen des Inaktivierungsbehälters dividiert.

Die Masse von 1 l $N_2$ bei 0°C ( = 273 K) und 760 mm Hg ( = 1 atm = 1013 mbar) ist 1,2505 g, bzw. 1 g $N_2$ unter diesen Normalbedingungen hat ein Volumen von 0,800 l. Nach den Gesetzen von Gay-Lussac und Boyle-Mariotte gilt:

$$\frac{p_1 \cdot v_1}{T_1} = \frac{p_2 \cdot v_2}{T_2} \qquad \text{Gleichung 2}$$

Mit den entsprechenden Werten von $p_1$, $v_1$, $T_1$ und $p_2$, $v_2$, $T_2$ für Normalbedingungen und 60°C erhält man:

$$\frac{1013 \cdot 0,800}{273} = \frac{p_{N_2/60} \cdot v_{1gN_2/60}^*}{333} \qquad \text{Gleichung 3}$$

$* = $ Volumen von 1 g $N_2$ bei 60°C)

Der gesuchte Wert $v_{1gN_2/60}$ in Liter (Spalte 12) wird durch Umformen von Gleichung 3 und Einsetzen des Wertes aus Spalte 10 erhalten.

$$v_{1gN_2/60} = 2,97 \cdot \frac{333}{1100} = 0,8991 \ (\text{Spalte } 12)$$

Die Division der Werte in Spalte 11 und 12 liefert den gesuchten Ausdruck $g_{H_2O}$ pro l $N_2$ bei 60°C in der

5

Gasphase (Spalte 13). Die Multiplikation mit dem Volumen des Inaktivierungsbehälters (Spalte 5) liefert das gewünschte Ergebnis: g Wasser in Form von Wasserdampf im Inaktivierungsgefäß bei 60°C. Bei dieser Berechnung wurde der Fehler durch das Volumen des Lyophilisates vernachlässigt.

Daraus ergibt sich, daß bei 60°C im Inaktivierungsbehälter lediglich etwa 4 % der gesamten vorgelegten Wassermenge in der Gasphase vorliegen, daß also auch bei erhöhter Temperatur der größte Teil des Wassers am Lyophilisat physikalisch gebunden verbleibt.

Der Äthanolgehalt in der Atmosphäre kann bestimmt werden, indem nach Erreichen der Temperatur von 60°C und Einstellen eines Gleichgewichtes im Behälter zwischen Feststoffen und Atmosphäre mehrere Gasproben von je 20 ml mittels einer Injektionsspritze entnommen und gaschromatographisch analysiert werden.

Es wurde gefunden, daß der Äthanolgehalt der Atmosphäre 93,2 mg pro l Behältervolumen betrug, woraus ersichtlich ist, daß nahezu 40 % der vorhandenen Äthanolmenge nicht mit Proteinen assoziiert, sondern frei in der Gasphase vorhanden ist.

Nach der oben erwähnten zehnstündigen Erhitzung auf 60°C wurde das inaktivierte Bulkpulver entnommen und bei einer Temperatur von +2 bis +8°C gelagert. Die Bestimmung der Faktor VIII-Aktivität vor und nach der Hitzebehandlung ergab eine Restaktivität von 0,71 (71 % Ausbeute an Faktor VIII).

Zur Prüfung des Virus-Inaktivierungseffektes wurde die Kinetik der erfindungsgemäßen Inaktivierung von verschiedenen Modellviren in einem Parallelversuch untersucht. Dabei wurde auch die nach der Inaktivierung vorhandene Restaktivität an Faktor VIII bei vergleichbarer Hitzebehandlung an virusfreien Proben bestimmt.

Zur Verfolgung der Inaktivierungskinetik wurde ein Alligot des vorstehend beschriebenen Faktor VIII-Materials in Form einer wässerigen Lösung mit einer Vaccinia-Virussuspension in Zellkulturmedium TCM 199 bzw. mit virusfreiem TCM 199 versetzt, in mehrere Flaschen verfüllt und gefriergetrocknet. Das gefriergetrocknete Faktor VIII-Konzentrat wurde auf einen Wassergehalt von 0,20 (20 Gew.%) eingestellt, mit trockenem Stickstoff, wie vorstehend beschrieben, "begast" und in den geschlossenen Fläschchen während 10 Stunden auf 60°C erhitzt. Nach 1, 3 und 10 Stunden wurde der Virustiter bestimmt, wobei jeweils das hitzebehandelte Präparat in Wasser gelöst und mit isotoner Kochsalzlösung im Verhältnis 1 : 10 seriell verdünnt wurde. Der Titer des Virus wurde durch Bewertung des zytopathischen Effektes auf sensitive Vero-Zellen in der Mikrotiterplatte bestimmt. Die Ergebnisse wurden nach statistischer Behandlung der Auswertung entsprechend der Formel von Reed und Muench als $TCID_{50}$ ausgedrückt (Reed J.L. and H.Muench; Amer.J.Hyg. 27 493, (1938)).

In gleicher Weise wurde die Kinetik der Virusinaktivierung bei Anwendung des erfindungsgemäßen Verfahrens gegenüber dem gleichen Vaccinia-Virus untersucht, wobei nach Einstellung des Wassergehaltes von 0,20 (20 Gew.%) und "Stickstoff-Begasung" eine Äthanolatmosphäre im Fläschchen von 0,25 g/l Behältervolumen aufrechterhalten wurde.

Unter sonst gleichen Bedingungen zeigt die Tabelle II den bedeutenden Fortschritt der erfindungsgemäßen Arbeitsweise unter Verwendung einer Alkoholatmosphäre gegenüber der aus der US-A- 4,640,834 bekannten Arbeitsweise (St.d.T.).

Als weiteres Modellvirus zur Überprüfung der erfindungsgemäßen Inaktivierungskinetik wurde Bakteriophage X 174 eingesetzt und mit dem Stand der Technik verglichen. Hierbei ist hervorzuheben, daß sowohl Vaccinia-Virus als auch Bakteriophage X 174 außerordentlich hitzestabile Viren sind. Die mit diesen Modellviren erhaltenen Ergebnisse sind geeignet, Schlußfolgerungen zu ziehen auf die Wirksamkeit des erfindungsgemäßen Verfahrens gegenüber Viren, die in Blutplasmaprodukten vorkommen können und deren Inaktivierung für die Sicherheit der Produkte bei der Anwendung am Menschen wichtig ist, wie z.B. HIV (Erreger von AIDS), Hepatitis B-Viren, Hepatitis NANB-Viren, CMV, EBV.

Tabelle II

Kinetik der Virusinaktivierung und Faktor VIII-Aktivität beim Erhitzen auf 60°C einer gefriergetrockneten Faktor VIII-Präparation

| | Wasser-gehalt | Äthanol (g/l) | Vaccinia Virustiter ($TCID_{50}$) nach Erhitzen während | | | | Restaktivität Faktor VIII nach Erhitzen während | | | | |
|---|---|---|---|---|---|---|---|---|---|---|---|
| | | | 0 | 1 | 3 | 10 | 0 | 1 | 3 | 10 | Stunden |
| eg. Kom-bination org. Ver-bindung + Wasser | 0,20 | 0,25 | $10^{4,4}$ | $10^{3,5}$ | $10^{1,2}$ | $<10^1$ | 0,92 | 0,82 | 0,80 | 0,65 | |
| Kontroll-versuch ohne org. Verbin-dung | 0,20 | 0 | $10^{4,6}$ | $10^{4,2}$ | $10^{3,7}$ | $10^{1,4}$ | 1 | 0,97 | 0,96 | 0,87 | |

| | Wasser-gehalt | Äthanol (g/l) | Bakteriophage X 174-Virustiter (PFU) nach Erhitzen während | | | | |
|---|---|---|---|---|---|---|---|
| | | | 0 | 1 | 3 | 10 | Stunden |
| eg. Kom-bination org. Ver-bindung + Wasser | 0,20 | 0,25 | $10^{5,9}$ | $10^{3,3}$ | $10^{1,8}$ | 0 | |
| Kontroll-versuch ohne org. Verbin-dung | 0,20 | 0 | $10^{5,8}$ | $10^{4,1}$ | $10^{2,8}$ | $10^{1,5}$ | |

EP 0 324 729 A1

Aus den in der Tabelle II niedergelegten Ergebnissen ist im einzelnen zu ersehen, daß bei dem Kontrollversuch -entsprechend der Arbeitsweise nach der US-A - 4,640,834 -unter Verwendung von Vaccinia Virus als Modellvirus bei einem Wassergehalt von 0,20 der zu prüfenden Probe der Virustiter nach zehnstündiger Erhitzung $10^{1,4}$ betrug. Demgegenüber wurde dieser Wert bei erfindungsgemäßer Arbeitsweise, nämlich bei Aufrechterhaltung einer äthanolischen Atmosphäre während der Inaktivierung schon nach drei Stunden erreicht. Bei Verwendung einer äthanolischen Atmosphäre gemäß der Erfindung war nach zehnstündiger Erhitzung das Vaccinia Virus nicht mehr nachweisbar, während ohne Äthanolatmosphäre nach zehnstündiger Erhitzung der Virustiter noch $10^{1,4}$ betrug.

Gleichermaßen überlegene Ergebnisse gegenüber dem Kontrollversuch ohne Äthanol wurden auch bei Verwendung von Bakteriophagen X 174 als Modellvirus festgestellt, d.h. der bei der Inaktivierung ohne Alkohol nach 10stündiger Inaktivierungsdauer festgestellte Virustiter von $10^{1,5}$ wurde bei der erfindungsgemäßen Arbeitsweise mit Alkohol schon nach 3 Stunden erreicht. Nach 10stündiger Erhitzung war nach dem erfindungsgemäßen Verfahren kein Virus nachweisbar.

Die Faktor VIII-Aktivitätsbestimmung vor und nach der Hitzebehandlung erfolgte mit Hilfe des 2-Stufen-Tests. Die daraus berechnete Restaktivität an Faktor VIII nach der Erhitzung war in allen Fällen ausreichend.

Beispiel 2:

Eine die Gerinnungsfaktoren II, IX und X enthaltende Präparation wurde nach der in Vox Sang. 33, 37-50 (1977) beschriebenen Methode aus menschlichem Plasma durch Adsorption an DEAE-Sephadex, Waschen des Ionenaustauschers und Elution des partiellen Prothrombinkomplexes gewonnen.

Das Eluat wurde dialysiert, gefriergetrocknet und daraus eine wässerige Lösung des partiellen Prothrombinkomplexes mit einem Gehalt von 40 mg Protein/ml bereitet. Jeweils 2 ml dieser Lösung wurde mit einer Suspension von Vaccinia-Virus in Zellkulturmedium bzw. mit isotoner Kochsalzlösung gemischt, in Fläschchen abgefüllt und gefriergetrocknet. Alle Proben wurden auf einen Wassergehalt von 0,15 eingestellt, und der Hälfte aller Proben wurden 0,25 g Äthanol pro l Behältervolumen zugesetzt. Die geschlossenen Behälter mit gefriergetrocknetem, partiellem Prothrombinkomplex - mit und ohne Virus - wurden bei einer Temperatur von 70°C verschieden lang erhitzt. Je drei Proben wurden vor dem Erhitzen sowie nach einer Stunde und nach drei Stunden Erhitzen zur Messung des Virustiters einerseits und der Faktor IX-Restaktivität und des Wassergehaltes andererseits entnommen. Die Bestimmung des Virustiters erfolgte wie in Beispiel 1 beschrieben.

Die Bestimmung der Faktor IX-Aktivität erfolgte durch Zusatz der zu testenden Probe zu einem Faktor IX-Mangelplasma und Bestimmung der aktivierten partiellen Thromboplastinzeit (1-Stufen-Test). Die Bestimmung des Wassergehaltes wurde wie in Beispiel 1 durchgeführt.

Die nach erfolgter Hitzebehandlung des feuchten Lyophilisates mit Alkoholdampf bestimmten Virustiter sind der Tabelle III zu entnehmen, woraus zu sehen ist, daß die Nachweisgrenze des Virus schon nach drei Stunden erreicht wurde, wobei die Faktor IX-Restaktivität in ausreichendem Maße erhalten blieb. Im Vergleich dazu ist zu ersehen, daß nach Erhitzen der feuchten, partiellen ProthrombinkomplexProben ohne Alkoholzusatz der Virustiter nur geringfügig abnahm.

Tabelle III

Kinetik der Virusinaktivierung beim Erhitzen eines partiellen
Prothrombinkomplexes

| | Wasser-gehalt | Tempera-tur °C | Äthanol-gehalt g/l | Vaccinia-Virustiter (TCID$_{50}$) nach Erhitzen während 0    1    3 h | | |
|---|---|---|---|---|---|---|
| eg. Kombination org. Verbindung + Wasser | 0,15 | 70 | 0,25 | $10^{6,5}$ | $10^{1,1}$ | $<10^{1}$ |
| Kontrollversuch ohne organische Verbindung | 0,15 | 70 | 0 | $10^{5,9}$ | $10^{5,2}$ | $10^{4,1}$ |

Beispiel 3:

Es wurden zwei Ansätze einer wässerigen Lösung einer Faktor VIII-hältigen Präparation nach der AT-B - 379.510 hergestellt, mit Calciumionen in Form von Calciumchlorid und einer Vaccinia Virussuspension versetzt und gefriergetrocknet. einmal betrug die Menge an Calciumchlorid 18 mg, das andere Mal 27,5 mg pro g des trockenen Lyophilisates. Die gefriergetrockneten Faktor VIII-Konzentrate wurden auf einen Wassergehalt von 0,20 eingestellt, mit Stickstoff, wie zuvor beschrieben, "begast" und in den geschlossenen Fläschchen 10 Stunden lang auf 60°C erhitzt. Nach 1, 3 und 10 Stunden wurde, wie in Beispiel 1 beschrieben, der Virustiter bestimmt. An gleichartig behandelten, jedoch virusfreien Proben, wurde die Faktor VIII-Aktivität bestimmt. In gleicher Weise wurde der zeitliche Verlauf des Virustiters und der Faktor VIII-Aktivität bei Anwendung des erfindungsgemäßen Verfahrens untersucht, wobei nach Einstellung des Wassergehaltes auf 0,20 eine Äthanolatmosphäre im Fläschchen von 0,4 g pro l Behältervolumen aufrechterhalten wurde.

Wie man den in Tabelle IV zusammengestellten Ergebnissen entnehmen kann, führt die Kombination der Einwirkung von Alkoholdampf auf das befeuchtete Lyophilisat zu einer gegenüber dem Stand der Technik erheblich verbesserten Wirkung der Virusinaktivierung. Gleichzeitig bewirkt die Anwesenheit von Calciumionen eine Stabilisierung der Faktor VIII-Aktivität gegenüber der Einwirkung von Äthanol.

Tabelle IV
Kinetik der Virusinaktivierung und der Faktor VIII-Aktivität beim Erhitzen einer
gefriergetrockneten Faktor VIII-Präparation auf $60^\circ C$ in Anwesenheit von Calciumionen

| | Wasser-gehalt | Äthanol (g/l) | Calcium-chlorid mg/g Pulver | Vaccinia Virustiter $(TCID_{50})$ nach Erhitzen während | | | | | Restaktivität Faktor VIII nach Er-hitzen während | | | |
|---|---|---|---|---|---|---|---|---|---|---|---|---|
| | | | | 0 | 1 | 3 | 10 | 30h | 0 | 1 | 3 | 10 h |
| eg. Kombi-nation org. Verbindung + Wasser + Ca | 0,20 | 0,4 | 27,5 | $10^{5,0}$ | $10^{4,9}$ | $10^{3,1}$ | $<10^1$ | -* | 1 | 0,9 | 0,9 | 0,8 |
| Kontrollver-such ohne organische Verbindung | 0,20 | 0 | 18 | $10^{5,5}$ | $10^{5,5}$ | $10^{5,3}$ | $10^{4,0}$ | -* | 1 | -*) | 0,9 | 0,8 |
| Kontroll-versuch ohne Wasser$^x$ und ohne Ca | 0,02 | 0,63 | 0 | $10^{3,8}$ | -*) | -*) | $10^{3,3}$ | $10^{2,9}$ | 1 | -*) | -*) | 0,85 |
| Kontroll-versuch ohne org. Verb. und ohne Ca | 0,20 | 0 | 0 | $10^{5,5}$ | $10^{5,3}$ | $10^{5,1}$ | $10^{4,4}$ | -* | 0,9 | -*) | 0,8 | 0,7 |

*) nicht gemessen
$^x$ "ohne Wasser" bedeutet einen Wassergehalt unter 0,05 (5 Gew. %)

EP 0 324 729 A1

Beispiel 4:

Es wurde eine wässerige Lösung der in Beispiel 1 beschriebenen Faktor VIII-Präparation hergestellt. Jeweils 2 ml dieser Lösung wurden mit einer Suspension von Vaccinia-Virus in Zellkulturmedium bzw. mit isotoner Kochsalzlösung gemischt, in Fläschchen abgefüllt und gefriergetrocknet. Die Proben wurden auf die in der Tabelle V angegebenen Wassergehalte eingestellt und mit den in der Tabelle V angegebenen Mengen verschiedener organischer Verbindungen versetzt. Die geschlossenen Fläschchen wurden bei einer Temperatur von 60°C verschieden lang erhitzt.

Die Resultate hinsichtlich Virusinaktivierung sowie Faktor VIII-Restaktivität sind der Tabelle V zu entnehmen. Daraus ist klar ersichtlich, daß bei den Kontrollproben (Behandlung mit der jeweiligen organischen Verbindung bei einem Wassergehalt des Pulvers unter 0,05 bzw. unter 5 Gew.%) der Vaccinia-Virustiter nach 30stündiger Behandlung um weniger als eine log-Stufe reduziert wurde (Rest-Titer ca. $10^{3,5}$), während bei den Proben nach dem erfindungsgemäßen Verfahren (Wassergehalt des Pulvers über 0,05 bzw. über 5 Gew.%) die jeweilige organische Verbindung bewirkte, daß der Vaccinia-Virustiter nach 1 bis 10 Stunden Erhitzung nicht mehr nachweisbar war (Virus-Titer kleiner als $10^1$). Die Faktor VIII-Restaktivität nach der Erhitzung war in allen Fällen ausreichend.

Tabelle V

Einfluß verschiedener organischer Verbindungen auf die Kinetik der Virusinaktivierung und der Faktor VIII-Aktivität beim Erhitzen einer gefriergetrockneten Faktor VIII-Präparation auf 60°C

| | Essigsäure-ethylester (g/l) | Wasser-gehalt | Vaccinia Virustiter ($TCID_{50}$) nach Erhitzen während | | | | | Restaktivität Faktor VIII nach Erhitzen während | | | |
|---|---|---|---|---|---|---|---|---|---|---|---|
| | | | 0 | 1 | 3 | 10 | 30 | 0 | 1 | 3 | 10 h |
| eg. Kombination org. Verbindung + Wasser | 4,8 | 0,10 | $10^{3,0}$ | -*) | $10^{2,7}$ | $<10^1$ | -*) | 0,90 | 0,80 | 0,66 | 0,53 |
| eg. Kombination org. Verbindung + Wasser | 0,36 | 0,25 | $10^{3,1}$ | $<10^1$ | $<10^1$ | $<10^1$ | -*) | 1,02 | 0,93 | 0,68 | 0,60 |
| Kontrollversuch ohne Wasser[x] | 0,72 | 0,02 | $10^{4,3}$ | -*) | -*) | $10^{3,9}$ | $10^{3,4}$ | 1 | -*) | -*) | 0,90 |

*) nicht bestimmt

x) = "ohne Wasser" = Wassergehalt unter 0,05 (5 Gew.%)

Fortsetzung der Tabelle V

| | Diethyl-ether (g/l) | Wasser-gehalt | Vaccinia Virustiter ($TCID_{50}$) nach Erhitzen während | | | | | Restaktivität Faktor VIII nach Erhitzen während | | | |
|---|---|---|---|---|---|---|---|---|---|---|---|
| | | | 0 | 1 | 3 | 10 | 30 | 0 | 1 | 3 | 10 h |
| eg. Kombination org. Verbindung + Wasser | 0,43 | 0,25 | $10^{3,2}$ | $10^{2,6}$ | $10^{1,6}$ | $<10^1$ | – | 0,98 | 0,89 | 0,74 | 0,47 |
| Kontrollversuch ohne Wasser[x] | 0,57 | 0,02 | $10^{4,3}$ | – | – | $10^{3,6}$ | $10^{3,5}$ | 1 | – | – | 1,00 |

| | Dimethyl-formamid (g/l) | Wasser-gehalt | Vaccinia Virustiter ($TCID_{50}$) nach Erhitzen während | | | | | Restaktivität Faktor VIII nach Erhitzen während | | | |
|---|---|---|---|---|---|---|---|---|---|---|---|
| | | | 0 | 1 | 3 | 10 | 30 | 0 | 1 | 3 | 10 h |
| eg. Kombination org. Verbindung + Wasser | 0,4 | 0,08 | $10^{3,1}$ | – | $10^{2,9}$ | $<10^1$ | – | 1,00 | – | 0,69 | 0,55 |
| Kontrollversuch ohne Wasser[x] | 0,8 | 0,02 | $10^{4,4}$ | – | – | $10^{3,8}$ | $10^{3,1}$ | 1 | – | – | 0,88 |

EP 0 324 729 A1

Fortsetzung der Tabelle V

|  | Toluol (g/l) | Wasser-gehalt | Vaccinia Virustiter ($TCID_{50}$) nach Erhitzen während | | | | | Restaktivität Faktor VIII nach Erhitzen während | | | |
|---|---|---|---|---|---|---|---|---|---|---|---|
|  |  |  | 0 | 1 | 3 | 10 | 30 | 0 | 1 | 3 | 10 h |
| eg. Kombination org. Verbindung + Wasser | 0,35 | 0,21 | $10^{3,2}$ | $10^{2,9}$ | $10^{1,9}$ | $<10^1$ | – | 1,00 | 0,91 | 0,77 | 0,55 |
| Kontrollversuch ohne Wasser[x] | 0,7 | 0,02 | $10^{4,4}$ | – | – | $10^{3,5}$ | $10^{3,5}$ | 1 | – | – | 0,94 |

|  | Chloroform (g/l) | Wasser-gehalt | Vaccinia Virustiter ($TCID_{50}$) nach Erhitzen während | | | | | Restaktivität Faktor VIII nach Erhitzen während | | | |
|---|---|---|---|---|---|---|---|---|---|---|---|
|  |  |  | 0 | 1 | 3 | 10 | 30 | 0 | 1 | 3 | 10 h |
| eg. Kombination org. Verbindung + Wasser | 0,9 | 0,25 | $10^{2,9}$ | $10^{1,9}$ | $<10^1$ | $<10^1$ | – | 0,90 | 0,89 | 0,78 | 0,59 |
| Kontrollversuch ohne Wasser[x] | 1,2 | 0,02 | $10^{4,0}$ | – | – | $10^{3,6}$ | $10^{3,4}$ | 1 | – | – | 0,85 |

EP 0 324 729 A1

Fortsetzung der Tabelle V

| n-Heptan (g/l) | Wasser-gehalt | Vaccinia Virustiter ($TCID_{50}$) nach Erhitzen während | | | | | Restaktivität Faktor VIII nach Erhitzen während | | | |
|---|---|---|---|---|---|---|---|---|---|---|
| | | 0 | 1 | 3 | 10 | 30 | 0 | 1 | 3 | 10 h |
| eg. Kombination org. Verbindung + Wasser | 0,4 | 0,25 | $10^{3,4}$ | $10^{1,6}$ | $<10^1$ | | – | 1,11 | 1,08 | 1,00 | 0,78 |
| Kontrollversuch ohne Wasserˣ | 0,55 | 0,02 | $10^{4,0}$ | – | – | $10^{3,5}$ | $10^{3,8}$ | 1 | – | – | 0,95 |

| 1,2-Diacet-oxiethan (g/l) | Wasser-gehalt | Vaccinia Virustiter ($TCID_{50}$) nach Erhitzen während | | | | | Restaktivität Faktor VIII nach Erhitzen während | | | |
|---|---|---|---|---|---|---|---|---|---|---|
| | | 0 | 1 | 3 | 10 | 30 | 0 | 1 | 3 | 10 h |
| eg. Kombination org. Verbindung + Wasser | 0,45 | 0,25 | $10^{3,0}$ | $<10^1$ | $<10^1$ | $<10^1$ | – | 1,07 | 1,03 | 0,74 | 0,56 |
| Kontrollversuch ohne Wasserˣ | 0,9 | 0,025 | $10^{4,0}$ | – | – | $10^{3,8}$ | $10^{3,0}$ | 1 | – | – | 0,89 |

15

Fortsetzung der Tabelle V

| | Aceton (g/l) | Wassergehalt | Vaccinia Virustiter (TCID$_{50}$) nach Erhitzen während | | | | | Restaktivität Faktor VIII nach Erhitzen während | | | |
|---|---|---|---|---|---|---|---|---|---|---|---|
| | | | 0 | 1 | 3 | 10 | 30 | 0 | 1 | 3 | 10 h |
| eg. Kombination org. Verbindung + Wasser | 0,5 | 0,15** | $10^{3,5}$ | $10^{3,2}$ | $10^{1,6}$ | $<10^1$ | – | 0,94 | 0,85 | 0,78 | 0,74 |
| Kontrollversuch ohne Wasser$^x$ | 0,64 | 0,025** | $10^{3,9}$ | – | – | $10^{3,5}$ | $10^{3,6}$ | 1 | – | – | 0,92 |

**) – Sollwert, Wasserbestimmung nach Karl Fischer nicht durchführbar

EP 0 324 729 A1

**Patentansprüche**

1. Verfahren zur Inaktivierung von vermehrungsfähigen filtrierbaren Krankheitserregern in Blutplasmaprodukten unter Anwendung erhöhter Temperatur, wobei die Blutplasmaprodukte in festem Zustand in einem geschlossenen Inaktivierungsbehälter in Gegenwart hydroxylgruppenhältiger Verbindungen hitzebehandelt werden, deren Konzentration bezogen auf die Blutplasmaprodukte mehr als 0,05 (5 Gew.%) beträgt, gekennzeichnet durch die Kombination,
- daß die Blutplasmaprodukte vor der Hitzebehandlung auf einen Wassergehalt von 0,08 bis 0,40 (8 bis 40 Gew.%) eingestellt werden - wobei das im System enthaltene Wasser während der Hitzebehandlung im wesentlichen physikalisch an den Blutplasmaprodukten gebunden bleibt - und
- daß während der Hitzebehandlung der Blutplasmaprodukte in der Gasphase des Behälters eine organische Verbindung aus der Reihe Äthanol, Essigsäureethylester, Diethylether, Dimethylformamid, toluol, Chloroform, n-Heptan, 1,2-Diacetoxiethan oder Aceton mit einer Konzentration von 0,01 bis 10 g der organischen Verbindung pro Liter Behältervolumen vorgesehen wird.

2. Verfahren nach Anspruch 1, dadurch gekennzeichnet, daß die Hitzebehandlung in Gegenwart von Calciumionen durchgeführt wird.

3. Verfahren nach Anspruch 1, dadurch gekennzeichnet, daß die Blutplasmaprodukte mit Wasserdampf befeuchtet werden, bis der gewünschte Feuchtigkeitsgehalt erreicht ist, die Blutplasmaprodukte in den Inaktivierungsbehälter eingebracht werden und anschließend die organische Verbindung in Gasform in den Behälter eingeleitet wird, um die gewünschte Konzentration einzustellen, worauf der geschlossene Behälter mit den Blutplasmaprodukten auf die Inaktivierungstemperatur gebracht und während der für die Virusinaktivierung erforderlichen Zeit erhitzt wird.

4. Verfahren nach Anspruch 3, dadurch gekennzeichnet, daß die Befeuchtung der Blutplasmaprodukte mit Wasserdampf unter Vakuum durchgeführt wird.

5. Verfahren nach einem oder mehreren der Ansprüche 1 bis 4, dadurch gekennzeichnet, daß die Hitzebehandlung bei einer Temperatur von 50 bis 80°C durchgeführt wird.

6. Verfahren nach einem oder mehreren der Ansprüche 1 bis 5, dadurch gekennzeichnet, daß die Behälteratmosphäre vor der Einleitung der organischen Verbindung - durch Verdrängung mit inertem Gas, insbesondere mit trockenem Stickstoff, sauerstofffrei gemacht wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP-A-0 159 311 (IMMUNO)<br>* Seite 6, Zeilen 19-26; Seite 7, Zeilen 6-22; Anspruch 8 *<br>----- | 1-6 | A 61 L 2/04 |
| | | | **RECHERCHIERTE SACHGEBIETE (Int. Cl.4)**<br><br>A 61 L |

Der vorliegende Recherchenbericht wurde für alle Patentansprüche erstellt

| Recherchenort | Abschlußdatum der Recherche | Prüfer |
|---|---|---|
| DEN HAAG | 18-04-1989 | PELTRE CHR. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)